# EUROPEAN PATENT APPLICATION

(11) **EP 4 834 359 A1**
(43) Date of publication of application: **04.11.2026**
(21) Application number: 24913241.6
(22) Date of filing: 12.11.2024
(51) Int. Cl.: A61K 38/10, A61P 25/24

(54) **PHARMACEUTICAL COMPOSITION FOR PREVENTING OR TREATING DEPRESSION COMPRISING GV1001**

(30) Priority: 28.12.2023 KR 20230194435; 11.11.2024 KR 20240159033
(71) Applicant: Gemvax & Kael Co., Ltd., Daejeon 34036 (KR)
(72) Inventor: KIM, Sang Jae, Seongnam-si, Gyeonggi-do 13461 (KR)
(74) Representative: dompatent
(86) International application number: PCT/KR2024/017792
(87) International publication number: WO 2025/143530

(57) **Abstract**

The present invention relates to a pharmaceutical composition for preventing or treating depression, and more specifically, to a pharmaceutical composition for preventing or treating depression, comprising a peptide having the amino acid sequence of SEQ ID NO: 1, whereby the composition reduces the concentration of stress hormones in the blood, alleviates various symptoms caused by depression, is safe for the human body and has minimal side effects including adverse reactions.

## Description

### CROSS-REFERENCE TO RELATED APPLICATION AND CLAIM OF PRIORITY

This application claims priority to Korean Patent Application No. 10-2023-0194435 filed on December 28, 2023 and Korean Patent Application No. 10-2024-0159033 filed on November 11, 2024 in the Korean Intellectual Property Office (KIPO), the entire disclosures of which are incorporated by reference herein.

### [Technical Field]

The present invention relates to a pharmaceutical composition for preventing or treating depression comprising GV1001.

### [Background Art]

Depression is one of the most common mental illnesses; according to a WHO report, as of 2020, approximately 3.5% of the global population, that is, approximately 280 million people, were found to experience depression.

In Korea, according to the 2021 Mental Health Status Survey conducted by the National Mental Health Center under the Ministry of Health and Welfare, the lifetime prevalence of depression was found to be 7.7%. In addition, the socioeconomic losses due to depression amount to 2.0525 trillion won and have been reported to show an increasing trend.

Selective reuptake inhibitors, such as selective serotonin reuptake inhibitors (SSRIs), serotonin-norepinephrine reuptake inhibitors (SNRIs), serotonin-norepinephrine-dopamine reuptake inhibitors (SNDRIs), norepinephrine reuptake inhibitors (NRIs), and norepinephrine-dopamine reuptake inhibitors (NDRIs), have been primarily used as treatments for depression.

Selective reuptake inhibitors (SRIs) described above are known to be relatively safe compared to monoamine oxidase inhibitors (MAOIs) or tetracyclic antidepressants (TeCAs), which are associated with serious side effects; however, they have been reported to still be accompanied by many side effects, including sexual dysfunction, emotional blunting, glaucoma, and an increased risk of suicide in children and adolescents.

Therefore, there is a need to develop a method capable of treating depression more safely and effectively.

Accordingly, the present inventors conducted intensive research to develop a method capable of preventing or treating depression more safely and effectively. As a result, the present inventors confirmed that, when safety-proven GV1001 was administered to a mouse model of depression, various symptoms caused by depression were improved and the concentration of corticosterone in blood was decreased, thereby completing the present invention.

### [Summary of Invention]

### [Problems to be Solved by Invention]

An object of the present invention is to provide a pharmaceutical composition for preventing or treating depression.

Another object of the present invention is to provide a kit for preventing or treating depression.

Yet another object of the present invention is to provide a health functional food for preventing or alleviating depression.

### [Means for Solving Problems]

1. A pharmaceutical composition for preventing or treating depression comprising a peptide having the amino acid sequence of SEQ ID NO: 1.
2. A kit for preventing or treating depression comprising: the pharmaceutical composition according to item 1; and instructions describing a method for preventing or treating depression.
3. The kit according to item 2, wherein the method for preventing or treating depression includes administering the pharmaceutical composition to a subject who has developed or is at risk of developing depression.
4. A health functional food for preventing or alleviating depression comprising a peptide having the amino acid sequence of SEQ ID NO: 1.

### [Advantageous effects]

As a result of administering the pharmaceutical composition of the present invention to a mouse model of depression, not only were improved behavioral patterns observed in behavioral evaluations, but the concentration of corticosterone in blood was also reduced. Accordingly, the pharmaceutical composition of the present invention is expected to be effective in the treatment, alleviation, or prevention of depression.

The pharmaceutical composition and health functional food of the present invention are expected to be useful for the prevention, alleviation, or treatment of depression by comprising GV1001 as an active ingredient, which has been proven to be safe for the human body and to exhibit no cytotoxicity or fatal side effects in numerous clinical trials.

### [Brief Description of Drawings]

FIG. 1 schematically illustrates the schedule for a behavioral evaluation experiment to confirm the therapeutic efficacy of GV1001 against depression.
FIG. 2 illustrates the experimental results according to Example 2(1).
FIG. 3 illustrates the experimental results according to Example 2(2).
FIG. 4 illustrates the experimental results according to Example 2(3).
FIG. 5 illustrates the experimental results according to Example 2(4).
FIG. 6 schematically illustrates the schedule for an experiment to confirm the preventive efficacy of GV1001 against depression.
FIG. 7 illustrates the experimental results according to Example 2(5).

### [Mode for Carrying out Invention]

The present invention provides a pharmaceutical composition for preventing or treating depression comprising a peptide having the amino acid sequence of SEQ ID NO: 1.

In the present invention, the peptide having the amino acid sequence of SEQ ID NO: 1 comprises a functional equivalent thereof. As used herein, the term "functional equivalent" refers to a peptide that exhibits substantially the same physiological activity as the peptide having the amino acid sequence of SEQ ID NO: 1.

Depression is a mental disorder accompanied by symptoms such as a depressed mood and avoidance of activities.

As used herein, the term "depression" may be used interchangeably with "depressive disorder" and is used as a collective term for major depressive disorder and dysthymia.

As used herein, the term "prevention" refers to any act of suppressing or delaying depression.

As used herein, the term "treatment" or "alleviation" refers to any action that alleviates or beneficially modifies the symptoms of a subject who has developed or is at risk of developing depression. Accordingly, a pharmaceutical composition for treating depression may be used interchangeably with "a therapeutic agent for depression" or "an antidepressant."

The present inventors confirmed that administering the pharmaceutical composition of the present invention to a mouse model of depression reduced the concentration of corticosterone in blood in the mouse. Corticosterone is a major corticosteroid hormone in rats and mice and corresponds to a type of stress hormone, whereas the major adrenal stress hormone in humans is cortisol.

In the present invention, the stress hormone refers to corticosterone or cortisol.

The pharmaceutical composition of the present invention may prevent, alleviate, or treat depression by reducing the concentration of stress hormones in blood of a subject.

The pharmaceutical composition of the present invention may alleviate various symptoms caused by depression, such as lethargy, depression, and anxiety.

As used herein, the term "subject" refers to all animals that have developed or may develop depression, such as livestock and mice, and may be mammals, including humans, for example.

The pharmaceutical composition of the present invention may include the active ingredient alone, or may be provided as a pharmaceutical composition further comprising one or more pharmaceutically acceptable carriers, excipients, or diluents.

The carrier, excipient, or diluent that may be included in the pharmaceutical composition of the present invention may include lactose, dextrose, sucrose, dextrin, maltodextrin, sorbitol, mannitol, xylitol, erythritol, maltitol, starch, gum acacia, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, or mineral oil, but is not limited thereto.

The present invention provides a kit for preventing or treating depression, which comprises the pharmaceutical composition for preventing or treating depression and instructions describing a method for preventing or treating depression.

In one embodiment, a method for preventing or treating depression may comprise administering the pharmaceutical composition of the present invention to a subject who has developed or is at risk of developing depression.

As used herein, the term "administration" means introducing a predetermined substance into a subject by an appropriate method.

The route of administration of the pharmaceutical composition of the present invention may be oral, intravenous, intramuscular, intraarterial, intramedullary, intrathecal, intracardiac, transdermal, subcutaneous, intraperitoneal, intranasal, enteral, topical, sublingual, or rectal administration, but is not limited thereto.

In one embodiment, the composition of the present invention may be administered orally or parenterally.

When the composition of the present invention is administered parenterally, it is preferable to select an administration method such as topical application, intraperitoneal injection, intrarectal injection, subcutaneous injection, intravenous injection, intramuscular injection, or intrathoracic injection, but is not limited thereto.

The pharmaceutical composition of the present invention may be a solid preparation for oral administration, such as a tablet, pill, powder, granule or capsule.

The pharmaceutical composition of the present invention may be a liquid preparation for oral administration, such as a suspension, a solution, an emulsion or a syrup.

The pharmaceutical composition of the present invention may be a preparation for parenteral administration, such as a sterile aqueous solution, a non-aqueous solution, a suspension, an emulsion, a lyophilized preparation or a suppository.

In one embodiment, a method for preventing or treating depression may include administering a pharmaceutically effective amount of the pharmaceutical composition of the present invention to a subject who has developed or is at risk of developing depression.

In the present invention, the "pharmaceutically effective amount" refers to an amount sufficient to treat depression at a reasonable benefit/risk ratio applicable to medical treatment.

The pharmaceutically effective amount of the pharmaceutical composition of the present invention may be determined according to factors such as the severity of depression, the activity of the pharmaceutical composition, the sensitivity of the subject or patient to the pharmaceutical composition, the time and route of administration, the excretion rate, the duration of treatment, and concomitant medication, as well as other factors well known in the medical field, and may be appropriately selected by a person skilled in the art.

The pharmaceutical composition of the present invention may be administered as a single therapeutic agent or in combination with other therapeutic agents, may be administered sequentially or simultaneously with conventional therapeutic agents, and may be administered in a single or multiple dosage regimen, which may be readily determined by a person skilled in the art.

The present invention provides a health functional food for preventing or alleviating depression including a peptide having the amino acid sequence of SEQ ID NO: 1.

The health functional food of the present invention refers to food manufactured and/or processed in various forms to provide functionality beneficial to the human body.

The health functional food of the present invention may be incorporated into various foods or medicines known in the art.

There is no particular limitation on the type of food in which the health functional food of the present invention may be included. For example, the health functional food of the present invention may be included in meat, sausages, bread, chocolate, candies, snacks, confectionery, pizza, ramen, other noodles, gum, dairy products including ice cream, various soups, beverages, teas, drinks, alcoholic beverages, and vitamin complexes.

The health functional food of the present invention encompasses all forms, including functional foods, nutritional supplements, health foods, and food additives, and may be manufactured in various forms according to conventional methods known in the art. For example, the health food may be manufactured in the form of a liquid drink for oral intake, or may be granulated, encapsulated, tableted into spherical pills, or powdered for consumption. It may also be manufactured in the form of a powder, capsule, soft capsule, tablet, gum, or adhesive-type liquid composition. In addition, the functional food may include beverages (including alcoholic beverages); fruits and processed products thereof (e.g., canned fruits, bottled fruits, jams, marmalades, etc.); fish, meat and processed products thereof (e.g., ham, sausages, corned beef, etc.); breads and noodles (e.g., udon, buckwheat noodles, ramen, spaghetti, macaroni, etc.); fruit juices, various drinks, cookies, taffy, dairy products (e.g., butter, cheese, etc.); edible vegetable oils, margarine, vegetable proteins, retort foods, frozen foods, herbal decoctions, and seasonings (e.g., soybean paste, soy sauce, sauces, etc.).

The health functional food of the present invention may further include ingredients commonly added during food manufacture, as long as they do not deviate from the ultimate purpose of the present invention, and may further include, for example, proteins, carbohydrates, fats, other nutrients, seasonings, and flavoring agents.

The health functional food of the present invention may additionally contain various nutritional supplements, vitamins, electrolytes, flavoring agents, coloring agents, pectic acids and their salts, alginic acid and its salts, organic acids, protective colloid thickeners, pH regulators, stabilizers, preservatives, glycerin, alcohol, and carbonation agents used in carbonated beverages, etc.

The health functional food of the present invention may contain fruit pulp for the production of natural fruit juice, fruit juice beverages, and vegetable beverages. These ingredients may be used independently or in combination.

Hereinafter, the present invention will be described in detail with reference to examples in order to specifically describe the present invention. However, the following examples are provided only to facilitate understanding of the present invention, and the scope of the present invention is not limited by the following examples.

### Example

### 1. Synthesis of GV1001

A peptide consisting of 16 amino acids having a structure represented by Formula 1 below, which was selected from human telomerase (hereinafter referred to as GV1001), was synthesized:

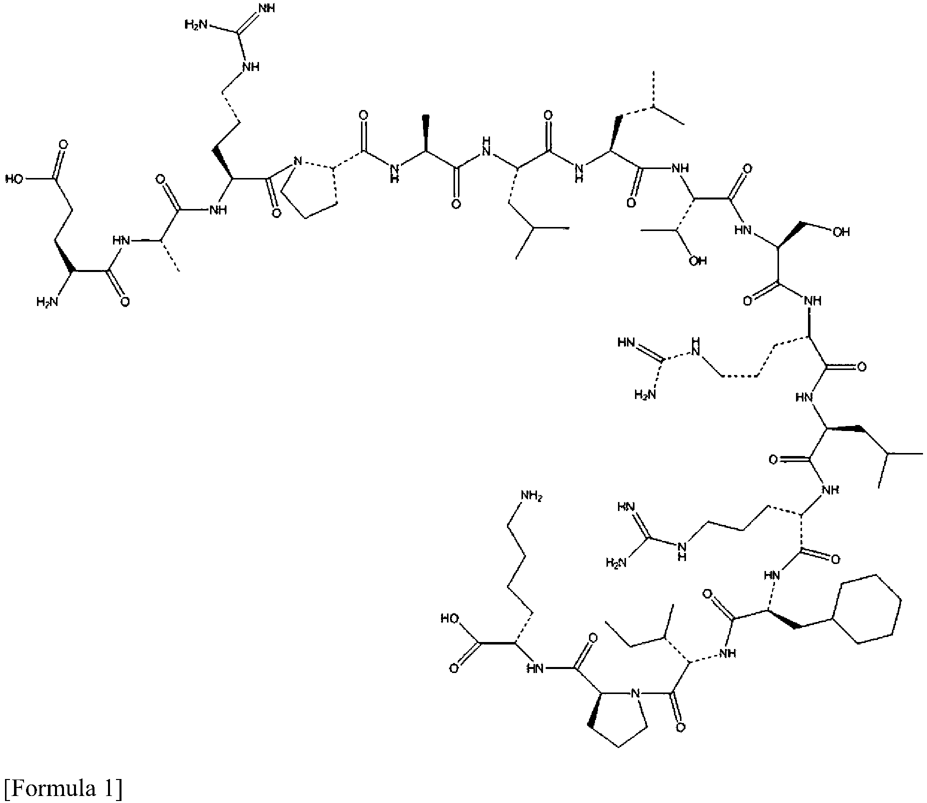

GV1001 was synthesized by coupling amino acids one by one starting from the C-terminus according to the Fmoc solid phase peptide synthesis (SPPS) method using ASP48S (Peptron, Inc., Daejeon, Korea).

All amino acid raw materials used for peptide synthesis were those in which the first amino acid of the C-terminus was attached to the resin. For example, the following compounds were used:
NH₂-Lys(Boc)-2-chloro-Trityl Resin
NH₂-Ala-2-chloro-Trityl Resin
NH₂-Arg(Pbf)-2-chloro-Trityl Resin

All amino acid raw materials used for peptide synthesis were those in which the N-terminus was protected with Fmoc, and the residues were protected with Trt, Boc, t-Bu (t-butyl ester), Pbf (2,2,4,6,7-pentamethyl dihydro-benzofuran-5-sulfonyl), or the like, which are removable under acidic conditions. For example, the following compounds were used:

Fmoc-Ala-OH, Fmoc-Arg(Pbf)-OH, Fmoc-Glu(OtBu)-OH, Fmoc-Pro-OH, Fmoc-Leu-OH, Fmoc-Ile-OH, Fmoc-Phe-OH, Fmoc-Ser(tBu)-OH, Fmoc-Thr(tBu)-OH, Fmoc-Lys(Boc)-OH, Fmoc-Gln(Trt)-OH, Fmoc-Trp(Boc)-OH, Fmoc-Met-OH, Fmoc-Asn(Trt)-OH, Fmoc-Tyr(tBu)-OH, Fmoc-Ahx-OH, Trt-Mercaptoacetic acid.

As coupling reagents, HBTU [2-(1H-benzotriazole-1-yl)-1,1,3,3-tetramethylaminium hexafluorophosphate], HOBt [N-hydroxybenzotriazole], and NMM [4-methylmorpholine] were used. Fmoc removal was conducted using 20% piperidine in DMF. The synthesized peptide was separated from the resin, while the protecting groups of the residues were removed using a cleavage cocktail [TFA (trifluoroacetic acid) / TIS (triisopropylsilane) / EDT (ethanedithiol) / H₂O = 92.5/2.5/2.5/2.5].

A peptide was synthesized by repeating a process of reacting each amino acid sequentially and washing the same with a solvent, followed by deprotection, using a solid-phase support to which the starting amino acid having a protecting group bound thereto was attached. The synthesized peptide was cleaved from the resin and purified by HPLC, and synthesis thereof was confirmed by MS, followed by freeze-drying.

A specific synthesis process of GV1001 is as follows:
(1) Coupling: Protected amino acid (8 equiv.) and coupling reagents, that is, HBTU (8 equiv.) / HOBt (8 equiv.) / NMM (16 equiv.), were dissolved in DMF and added to NH₂-Lys(Boc)-2-chloro-Trityl resin, followed by reaction at room temperature for 2 hours. The reaction product was washed with DMF, MeOH, and DMF in this order.
(2) Fmoc Deprotection: After adding 20% piperidine in DMF, the mixture was reacted twice at room temperature for 5 minutes, followed by washing with DMF, MeOH, and DMF in this order.
(3) Preparation of Basic Skeleton: A peptide basic skeleton was prepared by repeatedly performing the reactions of (1) and (2).
(4) Cleavage: A cleavage cocktail was added to the peptide resin after completion of synthesis to separate the peptide from the resin.
(5) Precipitation: After adding cooled diethyl ether to the obtained mixture, the mixture was centrifuged to precipitate the peptide.
(6) Purification and Powder Preparation: After purification by Prep-HPLC, the molecular weight was confirmed by LC/MS, followed by lyophilization to prepare a powder.

### 2. Confirmation of the Efficacy of GV1001 in an Animal Model of Depression

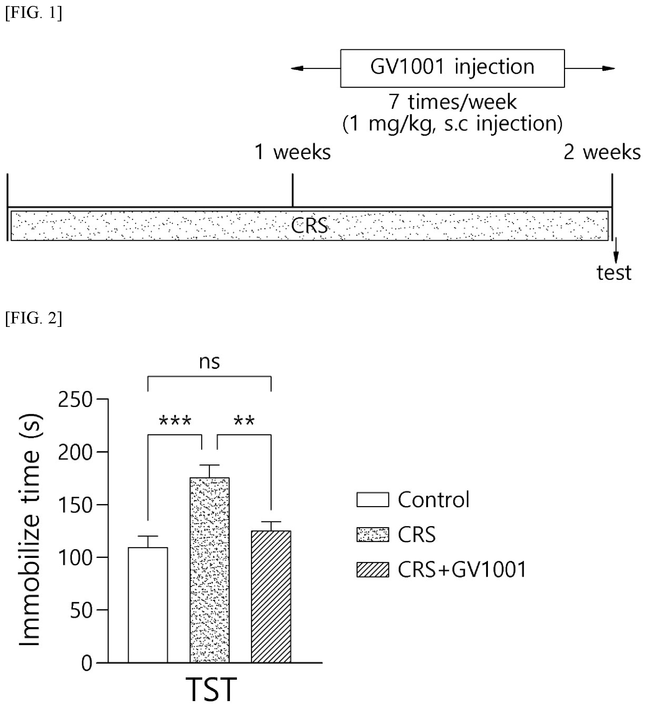

The present inventors established an animal model of depression using chronic restraint stress (CRS) among various methods for inducing depression, such as stress, transgenics, and neural injury. To this end, mice were restrained in 50 mL conical tubes for 3 hours daily for 2 weeks to prevent free movement, thereby inducing stress-induced depression. To confirm the therapeutic efficacy of GV1001 on symptoms of depression, GV1001 was administered daily via subcutaneous injection (SC injection) for one week starting from one week after the induction of depression, followed by conducting three types of behavioral assessments (FIG. 1).

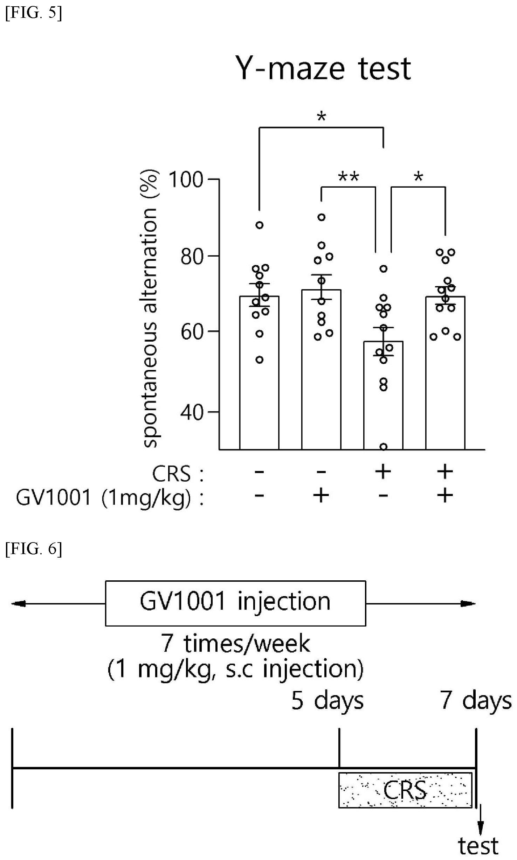

In addition, to confirm the prophylactic efficacy of GV1001 against depression, GV1001 was administered daily via subcutaneous injection for 7 days. After inducing depression by applying CRS for 2 days starting from the fifth day of GV1001 administration, blood samples were collected to measure changes in the concentration of stress hormones in blood (FIG. 6).

### (1) Confirmation of the Efficacy of GV1001 via Tail Suspension Test (TST)

Normal mice were designated as the 'Control,' and mice in which depression was induced by applying CRS for 3 hours daily for 2 weeks were designated as the 'CRS group.' A group in which CRS was applied for 2 weeks in the same manner as in the CRS group, but GV1001 was administered via subcutaneous injection at a dose of 1 mg/kg once daily for 1 week starting from one week after the initiation of CRS, was designated as the 'CRS+GV1001 group.'

As a result of conducting a TST on the three groups, the immobility time of the CRS group was significantly increased compared to the Control, confirming the occurrence of depression. Conversely, the immobility time of the CRS+GV1001 group was significantly reduced compared to the CRS group, confirming that GV1001 has a therapeutic effect on depression (FIG. 2).

### (2) Confirmation of the Efficacy of GV1001 via Sucrose Preference Test (SPT)

Mice were divided into three groups in the same manner as in Example 2(1), and a sucrose preference test, which is a behavioral assessment method for measuring anhedonia, a major symptom of depression, was performed. To this end, mice were exposed to water containing 1% sucrose for 24 hours and then fasted for 24 hours. Subsequently, the mice were simultaneously exposed to water containing 1% sucrose and regular water without sucrose, and the preference for sucrose was measured by comparing the intake amounts of the two types of water.

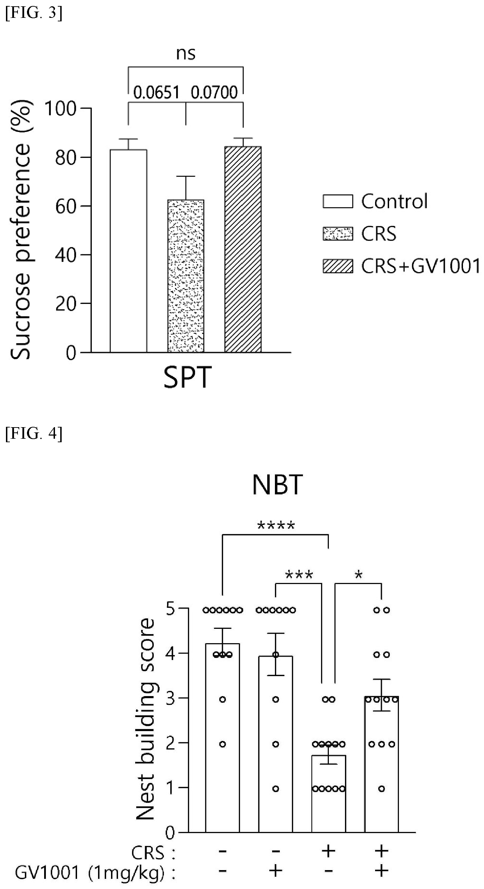

As a result, sucrose preference decreased in the CRS group compared to the Control, while sucrose preference in the CRS+GV1001 group increased compared to the CRS group, confirming the therapeutic effect of GV1001 on depression (FIG. 3).

### (3) Confirmation of the Efficacy of GV1001 via Nest-Building Test (NBT)

The following four groups of mice were prepared: a normal mouse group to which nothing was administered; a normal mouse group administered GV1001; a mouse group in which depression was induced by applying CRS for 3 hours daily for 2 weeks; and a group in which depression was induced by applying CRS for 3 hours daily for 2 weeks, but GV1001 was administered subcutaneously at a dose of 1 mg/kg once daily for 1 week starting from one week after the initiation of CRS (hereinafter referred to as the GV1001-administered depressed mouse group).

A nest-building test was conducted on these four groups to measure the degree of anxiety, which is one of the representative symptoms of depression. To this end, mice were individually placed in cages containing one piece of compressed cotton (Nestlet), and nest-building scores were measured using a five-point scale. According to the inherent characteristics of rodents, the greater the anxiety of the animals, the less likely they are to build nests.

As a result, there was no significant difference in nest-building scores between the normal mouse group and the normal mouse group administered GV1001, but the scores of the depression-induced mice decreased significantly. In the depression mouse group administered GV1001, the scores increased significantly compared to the depression-induced mouse group, confirming the effect of GV1001 on depression (FIG. 4).

### (4) Confirmation of the Efficacy of GV1001 via Y-maze Test

Four groups of mice were prepared in the same manner as in Example 2(3). A Y-maze test was performed on these four groups to measure the spatial working memory ability of the animals based on the natural behavior of rodents preferring to explore new environments rather than familiar environments. To this end, mice were allowed to freely explore three plastic arms arranged at 120-degree angles in a Y-shaped maze, and the number of times the mice entered an arm that they had not recently visited was measured.

As a result, there was no significant difference in the values (%) of spontaneous alternation between the normal mouse group and the normal mouse group administered GV1001; however, the values decreased significantly in the depression-induced mouse group. In the depression-induced mouse group administered GV1001, the values increased significantly compared to the depression-induced mouse group, confirming the effect of GV1001 on depression (FIG. 5).

### (5) Confirmation of the Efficacy of GV1001 Through Measurement of Stress Hormone Levels

The following four groups of mice were prepared: a normal mouse group to which nothing was administered; a normal mouse group administered GV1001; a mouse group in which depression was induced by applying CRS for 3 hours daily for 2 days; and a mouse group in which depression was induced by applying CRS for 3 hours daily for 2 days starting from the fifth day of administration, while GV1001 was administered via subcutaneous injection at a dose of 1 mg/kg once daily for 7 days (hereinafter referred to as the group of mice administered GV1001 before the induction of depression).

Blood samples were collected from these four groups, and the concentration of corticosterone in blood was measured.

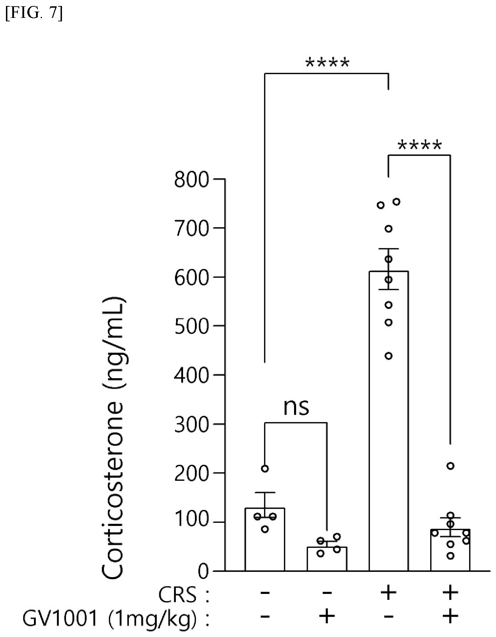

As a result, there was no significant difference in the concentration of corticosterone in blood between the normal mouse group and the normal mouse group administered GV1001; however, in the group of mice in which depression was induced, the concentration of corticosterone significantly increased. In the group of mice administered GV1001 before the induction of depression, the concentration of corticosterone in blood was statistically significantly reduced compared to the group of mice in which depression was induced. From these results, it was confirmed that GV1001 possesses not only a therapeutic effect but also a preventive effect against depression (FIG. 7).

## Claims

1. A pharmaceutical composition for preventing or treating depression comprising a peptide having the amino acid sequence of SEQ ID NO: 1.

2. A kit for preventing or treating depression comprising:
the pharmaceutical composition according to claim 1; and
instructions describing a method for preventing or treating depression.

3. The kit according to claim 2, wherein the method for preventing or treating depression comprises administering the pharmaceutical composition to a subject who has developed or is at risk of developing depression.

4. A health functional food for preventing or alleviating depression comprising a peptide having the amino acid sequence of SEQ ID NO: 1.
